# EUROPEAN PATENT SPECIFICATION

(11) **EP 3 215 065 B1**
(45) Date of publication and mention of the grant of the patent: **12.02.2020**
(21) Application number: 15828777.1
(22) Date of filing: 05.11.2015
(51) Int. Cl.: A61F 2/38, A61F 2/30

(54) **IMPROVED ADJUSTABLE MODULAR SPACER DEVICE FOR THE ARTICULATION OF THE KNEE**
VERBESSERTER ANPASSBARER MODULARER ABSTANDHALTER FÜR DIE BEUGUNG DES KNIES
ESPACEUR AMELIORE REGLABLE MODULAIRE POUR L'ARTICULATION DU GENOU

(30) Priority: 06.11.2014 IT RM20140646
(43) Date of publication of application: 13.09.2017
(73) Proprietor: Cossington Limited, 167-169 London Road Kingston Upon Thames Surrey KT2 6PT (GB)
(72) Inventor: Cappelletti, Ava, 47521 Cesena (IT)
(74) Representative: Dehns
(86) International application number: PCT/IT2015/000266
(87) International publication number: WO 2016/071938

(56) References cited:
- WO-A1-97/30661
- WO-A1-2013/041905
- DE-T2- 69 403 347
- US-A1- 2012 022 658

## Description

### TECHNICAL FIELD OF THE INVENTION

The present invention as defined in the claims concerns a knee spacer device, i.e. a device intended to be implanted temporarily at the joint area between the tibia and femur of a patient for maintaining the dimensions of such a zone.

### PRIOR ART

As known, it is sometimes necessary to temporarily remove joint prostheses, for example as a result of the onset of infections in the respective implant area. Moreover, an infected prosthesis or a prosthesis extracted from an infected area cannot be immediately replaced with a new prosthesis, but one must first treat and eliminate the infection with suitable antibiotic drugs.

It should then be noted that during the period required for the antibiotic treatment, the joint space wherein a new joint prosthesis is to be implanted must, of course, be kept unchanged, so as to prevent the shortening of the tissues, the atrophy of the joint and the loss of muscle tone.

In this regard, spacer devices are used, which are intended to be temporarily implanted at the joint area of a patient for replacing a prosthesis and for maintaining the size of the joint area itself before reimplanting a second prosthesis.

The spacer device has the function of maintaining the joint spaces as well as curing the bone infection by releasing amounts of antibiotic in the infected area. As regards the second function now indicated, the spacer can treat the ongoing infection by releasing antibiotic in a targeted manner and in infinitesimal quantities, while the application of even high doses of antibiotic, but with methods that do not involve the use of spacers, such as washing the infected area with solutions of high-dose antibiotics, does not allow obtaining the same results.

In fact, studies carried out in the field have revealed that the bone tissue absorbs in a concentrated manner all the (even a few) antibiotic molecules daily released by the spacer. This naturally occurs if the antibiotic is released by the spacer in contact with or near the bone tissue, in which case the amount of antibiotic reaches locally the effective concentration to eradicate the infection. For this reason, it is essential that the spacer extends across the whole infection area, this meaning that if the infected prosthesis is a long prosthesis, a long spacer will be used and, whether the infected prosthesis is of the short type, a short spacer will be used. Should a short spacer be placed where a long prosthesis was previously implanted, part of the bone would not be treated with antibiotic, letting in this way bacteria to proliferate.

Spacer devices are usually made of bone cement and with the appropriate size, extemporaneously or prior to their positioning, which greatly increases the duration of the implant operation and also sometimes involves the use of toxic substances.

Pre-formed spacer devices have also been proposed, which cannot be adapted from patient to patient, with the obvious consequence that, in most cases, it is very difficult to guarantee patients a good mobility of the joint before reimplanting the definitive prosthesis.

To this regard, WO2013041905A1 teaches a knee modular spacer provided with a femoral component, a tibial component and a shim or spacer, which can be located between tibial component and tibia. According to such piece of prior art, the tibial component includes a rod-like element such as to guarantee a correct mutual positioning between tibial component and shim.

### OBJECTS OF THE INVENTION

An object of the present invention is to provide a new knee spacer device as defined in the claims.

Another object of the present disclosure is to provide a knee spacer device that allows effective adjustments in the implant area.

Another object of the present disclosure is to provide a knee spacer device which suitable for self-adjusting to the respective implant area.

Another object of the present disclosure is to provide a new spacer device that can be implanted in a very quick and easy manner.

In accordance with an aspect of the invention a device according to claim 1 is provided.

The dependent claims refer to preferred and advantageous embodiments of the invention.

### BRIEF DESCRIPTION OF THE DRAWINGS

Other features and advantages of the invention will be more evident from the description of an embodiment of a device, shown by way of example in the accompanying drawings wherein:

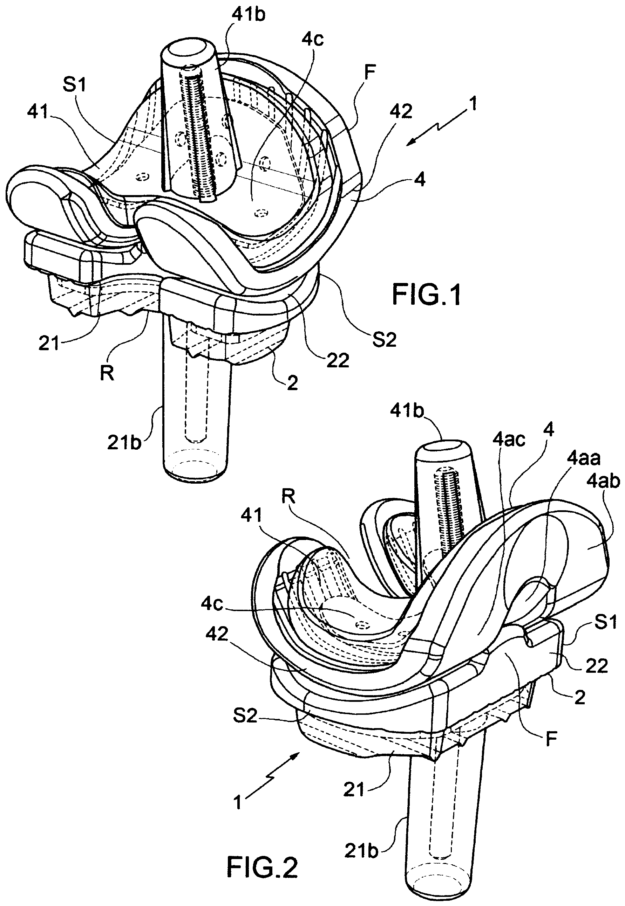

- figures 1 and 2 are perspective views from respective sides and with parts in transparency of a spacer device according to the present invention;

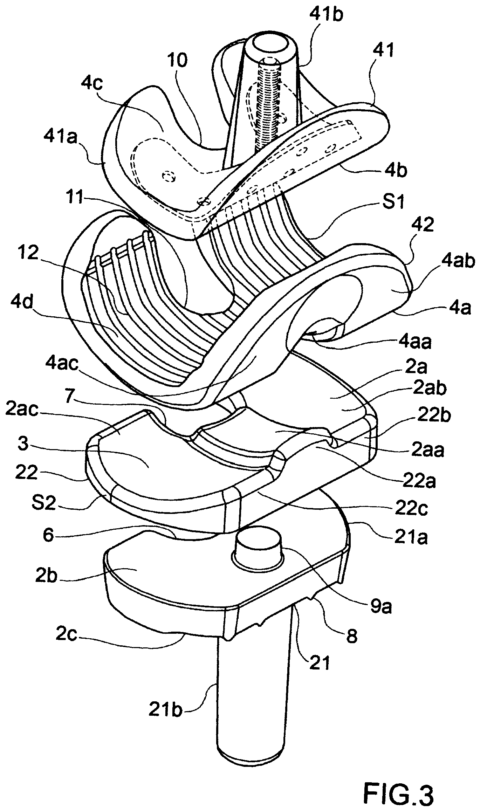

- figure 3 is a perspective view slightly from above, exploded and with parts in transparency of the device of figure 1;

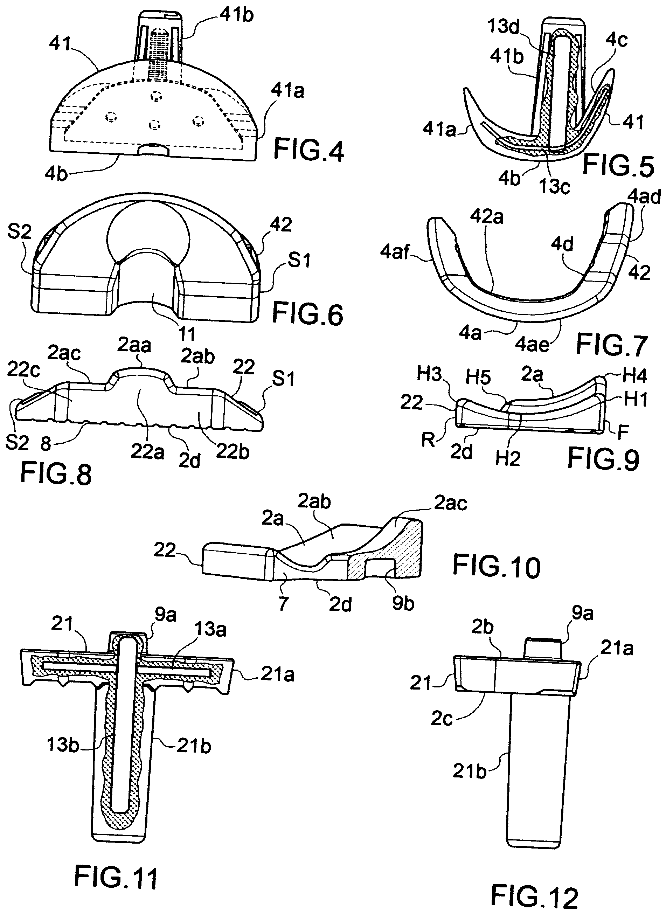

- figures 4 and 5 are a front view and a cross-section view of a first femoral component of a device according to the present invention;
- figures 6 and 7 are a front view and a side view of a second femoral component of a device according to the present invention;
- figures 8, 9 and 10 are a front view, a side view and a sectional view, respectively, of a second tibial component of a device according to the present invention;
- figures 11 and 12 are a front view and a cross-section view of a first tibial component of a device according to the present invention;
- figure 13 shoes the components of a kit for making a spacer device according to the present invention.

In the accompanying drawings, equal parts or components are identified by the same reference numbers.

### EMBODIMENTS OF THE INVENTION

With reference to the figures from 1 to 12, a knee spacer device 1 according to the present invention is shown, in particular an improved modular adjustable spacer device, if desired disposable, which is intended to be implanted temporarily at the joint area between the tibia and femur of a patient for the replacement of a knee joint prosthesis, for example infected or extracted from an infected area, and for the preservation of the size or spaces of the patient's joint area before implanting a new prosthesis, in particular after the treatment of the infected area.

In the present invention, there will be indicated as the front F of the device, the part thereof which, in use, is arranged at the front part of the knee wherein it is to be implanted, and as the rear R of the device, the part thereof which, in use, is arranged at the rear part of the knee wherein it is to be implanted. As regards the expressions front or fore end or portion or similar expressions, these indicate the ends of a component of the device proximal to the front F and distal from the rear R, while the expressions rear end or portion or similar expressions indicate the ends of a component of the device proximal to the rear R and distal from the front F.

More particularly, the spacer device 1 comprises a tibial unit 2 intended to be fixed to the tibia of a patient and having a first upper, in use, joint surface 2a substantially curved or ramp-shaped delimiting a substantially concave sliding seat 3.

The spacer device is then provided with a femoral unit 4 intended to be fixed to the femur of a patient and provided with a first lower, in use, joint face 4a substantially convex and intended to be positioned in the sliding seat 3 for the sliding engagement of the first upper joint surface 2a, thereby allowing for the joint, if desired a mutual angular or roto-translational displacement between tibial unit 2 and femoral unit 4 and hence, once the device has been implanted, between the tibia and the femur.

Moreover, the tibial unit 2 may comprise a first component 21 fixable to the tibia and distal from the first joint surface 2a, as well as a second component 22 adjustably fixable to the first component 21 in a plurality of operational positions and delimiting the first joint surface 2a, more particularly delimiting the entire first joint surface 2a. In alternative or in addition to this, the femoral unit 4 comprises a first component 41 fixable to the femur and distal from the first joint face 4a, as well as a second component 42 adjustably fixable to the first component 41 in a plurality of operational positions and delimiting the joint face 4a, more particularly delimiting the entire first joint face 4a.

In substance, the second tibial component 22, if provided, constitutes the component of the tibial unit 2 in (sliding or roto-translation) contact with the femoral unit 4 or with the second femoral component 42 of the latter, while the first tibial component 21 is fixed to the tibia and distal and not in contact with the femoral unit 4; moreover, the second tibial component 22 is not intended to come into contact with the tibia. As regards, instead, the second femoral component 42, if such a component is provided, it constitutes the component of the femoral unit 4 in (sliding or roto-translation) contact with the tibial unit 2 or with the second tibial component 22 of the latter, while the first femoral component 41 is fixed to the femur and distal and not in contact with the tibial unit 2; moreover, the second femoral component 42 is not intended to come into contact with the femur.

In the case wherein the tibial unit 2 comprises a first tibial component 21 fixable to the tibia as well as a second tibial component 22 adjustably fixable to the first tibial component 21, the first tibial component 21 may include a base portion 21a delimiting a second intermediate surface 2b, optionally substantially flat, facing towards the second tibial component 22 and a third connection surface 2c, optionally substantially flat, opposite to the second surface 2b and further comprises a first stem 21b, optionally removable, for example connectable by screwing to the base portion 21a, which rises from the third connection surface 2c of the base portion 21a and is intended to be implanted into the tibia of a patient. The second tibial component 22, instead, delimits on one side the first upper joint surface 2a and, on the other side, a fourth intermediate surface 2d intended to be abutted to and fixed against the second intermediate surface 2b of the first tibial component 21. The first stem 21b extends, in use, in a direction substantially longitudinal with respect to the tibial bone of the patient in which it is to be implanted.

If desired, the base portion 21a of the first tibial component 21 is substantially C-shaped and delimits a first channel 6 passing and extending from the second intermediate surface 2b to the third connection surface 2c, while the second tibial component 22 is substantially C-shaped and delimits a second channel 7 extending from the first joint surface 2a to the fourth intermediate surface 2d and is substantially aligned, in use, to the first channel 6.

The first joint surface 2a may include, from a side S1 to the other S2 of the device 1, a raised intermediate part 2aa, as well as two lowered lateral parts 2ab, 2ac placed one opposite to the other with respect to the intermediate part 2aa. To this end, the second tibial component 22 may be provided with an intermediate section with a greater thickness 22a, as well as two lateral sections with a lower thickness 22b, 22c placed one opposite to the other with respect to the intermediate section 22a. In this case, the second channel 7 may be delimited between a rear end of the raised section 22a and rear end lengths of the two lowered sections 22b, 22c. The raised intermediate part 2aa extends longitudinally to the sagittal plane of the knee.

The level or height, in use, of the first joint surface 2a, that is to say the distance of the joint surface 2a from the fourth intermediate surface 2d, may also present a maximum H1 at the front F of the device 1, thus decreasing gradually up to a minimum H2, at a central area and then increasing until reaching a level H3 greater than H2, but smaller than H1 at the rear R. If there are provided a raised intermediate part 2aa and two lowered lateral parts 2ab, 2ac, then the level of the lowered lateral parts 2ab, 2ac follows the pattern now described, while the raised intermediate part 2aa has a maximum level H4 at the front F of the device 1, greater than H1, and thus gradually decreases to a minimum H5, greater than H2, if desired without providing for a terminal section of an increasing height corresponding to that between H2 and H3 of the lowered lateral parts 2ab, 2ac.

A device according to the present invention may then comprise fixing means between the first component 21, 41 and the second component 22, 42 of the same unit (tibial or femoral), which include a first position or rest condition, wherein they connect without fixing the first component 21, 41 and the respective second component 22, 42, and a second position or fixing condition, wherein they fix in a position of the plurality of operational positions the second component 22, 42.

The fixing means may, for example, comprise at least one adhesive layer, such as bone cement between the first component 21, 41 and the second component 22, 42, which layer has a predetermined time of curing or solidification. The adhesive layer is initially intended to take hold or adhere on one side with the first component 21, 41 and, on the other, with the second component 22, 42 of the same unit (tibial or femoral), so as to connect without fixing the first 21, 41 and the second component 22, 42, as well as to harden or solidify successively so as to fix the second component 22, 42 in a position of the plurality of operational positions, so that it is possible to adjust the relative position between the first 21, 41 and respective second 22, 42 component in the period of curing or solidification of the adhesive layer.

In this regard, the second component 22, 42 may be moved with respect to the first component 21, 41 along a first front F-rear R direction, along a second direction from one side S1 to the other S2 and/or along a third direction orthogonal to the first and second direction, that is to say a direction orthogonal to the intermediate surfaces 2b, 2d.

The second intermediate surface 2b of the first tibial component 21 or the fourth intermediate surface 2d of the second tibial component 22 may then present at least one rough or jagged area 8, for example notched, in which, in use, there is provided or reported the adhesive layer, such as a layer of bone cement intended to take hold or adhere on one side with the first tibial component 21 and, on the other, with the second tibial component 22 for fixing them together.

Moreover, the device may then comprise reference means for the guided positioning with unstable constrain of a second component 22, 42 on the other component 21, 41 of the same unit (tibial or femoral), which are intended to allow positioning initially the second component 22, 42 on the other component 21, 41 without, however, locking them in place and without hindering the displacement of the second component 22, 42 in the plurality of operational positions, after which the fixing means are applied and when these are brought from the first to the second condition, the two components 21, 22 or 41, 42 are fixed in position. In this regard, from the first tibial component 21 or from the second tibial component 22, or rather from the second intermediate surface 2b or from the fourth intermediate surface 2d of the latter, a pin component 9a may extend, while in the other between the second tibial component 22 and the first tibial component 21, or rather in the other between the fourth intermediate surface 2d and the second intermediate surface 2b of the latter, at least one guided positioning recess 9b of the pin component 9a is formed. If desired, the engagement between the pin component 9a and positioning recess 9b is loose, that is to say that the pin component 9a has a width smaller than the recess 9b. The pin component 9a as well as the corresponding recess 9b, furthermore, may also be substantially cylindrical or hemispherical.

If the femoral unit 4 comprises a first femoral component 41 fixable to the femur as well as a second femoral component 42 adjustably fixable to the first femoral component 41, the first femoral component 41 presents a main part 41a delimiting a second intermediate face 4b facing the second femoral component 42 and a third connection face 4c opposite the second face 4b and also comprises a second stem 41b, also removable if desired, for example connectable by screwing, which rises from the third connection face 4c of the main part 41 and is intended to be grafted into the femur of a patient. The second femoral component 42 on one side delimits the first lower joint face 4a and, on the other side, a fourth intermediate face 4d intended to be abutted against the second intermediate face 4b of the first femoral component 41. If desired, the second intermediate face 4b and the fourth intermediate face 4d are substantially complementary.

The main part 41a of the first femoral component 41 may also be substantially C-shaped and delimit a third channel 10 extending from the second intermediate face 4b to the third connection face 4c, while the second femoral component 42 is substantially C-shaped delimiting a fourth channel 11 extending from the first joint face 4a to the fourth intermediate face 4d and substantially aligned with the third channel 10. In use, the channels 6, 7, 10 and 11 are substantially aligned or one after the other, so as to define a main rear light of the device.

Moreover, the main part 41a of the first femoral component 41 as well as the second femoral component 42 comprise a plate-like body substantially curved with convexity facing the tibial unit 2 and, in such a case, the first femoral component 41 is, in use, housed within a housing seat 42a delimited by the second femoral component 42 or rather by the fourth intermediate face 4d thereof.

The first joint face 4a may present a hollow central band 4aa, in use intended to slidably engage the raised intermediate part 2aa of the tibial unit, as well as two lateral enlarged bands 4ab, 4ac placed one on the opposite side to the other with respect to the hollow band 4aa and each intended to slidably engage a respective lowered part 2ab, 2ac. In such a case, the fourth channel 11 may be delimited between a rear end of the hollow central band 4aa and front end parts of the two lateral enlarged bands 4ab, 4ac. The hollow central band 4aa extends longitudinally to the sagittal plane of the knee.

If during the movement of the knee joint the spacer device 1 is subjected to stresses of lateral thrust, the raised intermediate part 2aa holds in place the hollow central band 4aa and thus the femoral element 4, ensuring a correct movement and good stability of the joint itself.

The first joint face 4a may also present a much greater extension in the front F-rear R direction compared to the first joint surface 2a. In this regard, from the front F to the rear R of the device, the first joint face 4a may include three curved sections with convexity, in use, facing the first joint surface and, more particularly, a first slightly curved section 4ad, then a second section 4ae with a curvature greater than the first section 4ad, and then a third slightly curved section 4af with a curvature corresponding to that of the first section 4ad.

More particularly, the radius of curvature of the second section 4ae may be smaller than the radius of curvature of the first joint surface 2a. Such radii of curvature are chosen in such a way as to allow combining each second femoral component 42 with each second tibial component 22 of a kit according to the present invention, which will be better described hereinafter.

If a hollow central band 4aa and two lateral enlarged bands 4ab, 4ac are provided, then the two lateral enlarged bands 4ab, 4ac follow the pattern now described, while the hollow central band 4aa presents a first slightly curved section 4ad and a second section 4ae, but, if desired, does not comprise a third section 4af.

The second intermediate face 4b of the first femoral component 41 or the fourth intermediate face 4d of the second femoral component 42 may include at least one rough or jagged area 12 and there is provided a layer of bone cement at the rough or jagged area 12 intended to take hold or adhere on one side with the first femoral component 41 and, on the other, with the second femoral component 42 for fixing them together.

The first tibial 21 or femoral 41 component, as well as the second tibial 22 or femoral 42 component are pre-formed and made of biologically compatible material, capable of being additivated and/or additivable with one or more pharmaceutical products, active and/or therapeutic ingredients intended to be released in the tissues of the patient adjacent to the device or rather in the above-mentioned joint area of a patient so as to treat the infection of the bone ends of the tibia and femur.

The biologically compatible material of the spacer device according to the invention may be porous, in particular include interconnected or non-interconnected pores.

The materials for the components of a spacer device according to the present invention can be chosen among: metals, metal alloys, metal-organics, ceramic, glass, plastic materials or a combination thereof.

The plastic materials can be selected among thermoplastic polymers, such as acrylic resins, polyethylene, polypropylene, polyester, etcetera, thermoformable polymers and other similar materials.

In one version of the invention, the biologically compatible material of which the components 21, 22, 41 and 42 of the spacer device 1 are made comprises a bone cement or polymethylmethacrylate.

The pharmaceutical products, active and/or therapeutic ingredients may include antibiotics, antiseptics, bacteriostatics, bactericides, antimycotics, chemotherapeutics, for example, gentamicin, vancomycin, etcetera, or other active ingredients.

The material of the spacer device, being porous, may be additivated with one or more pharmaceutical products, active and/or therapeutic ingredients at the production site, or subsequently by the physician when used on the patient, for example by impregnation.

Moreover, within the components of the device, in particular within the first tibial component 21 and/or the first femoral component 41, a stiffening core 13a, 13b, 13c, 13d, for example metallic, embedded within a biologically compatible material as mentioned above can be provided.

The device may then comprise removable connection means, such as clips between the tibial unit 2 and the femoral unit 4, which clips can serve in particular to limit the relative displacements of such units transversely to a front F-rear R direction or from a side S1 to the other S2 during bone implant operations.

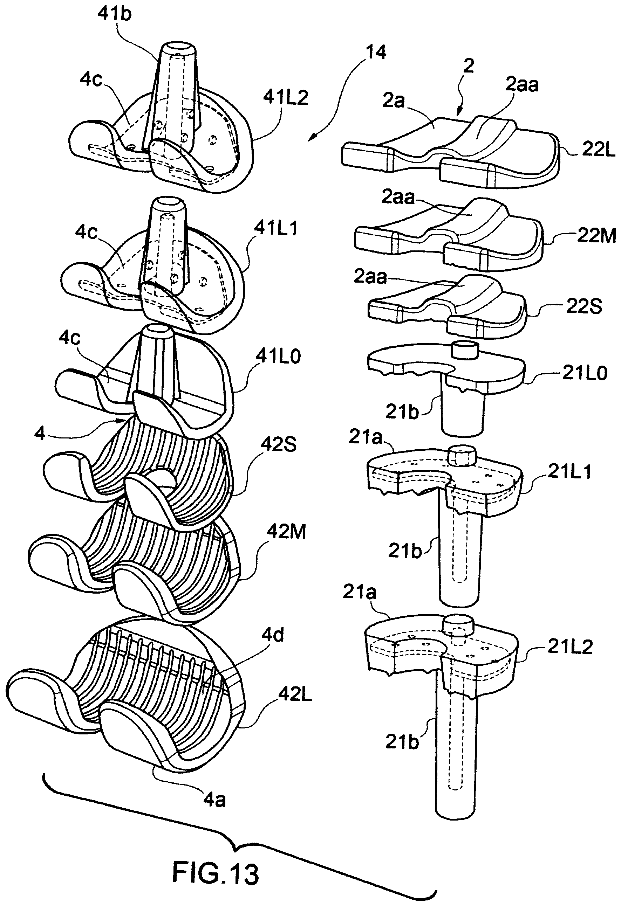

According to the present invention, a kit 14 (see figure 13) is also provided for the realisation of a knee spacer device according to the present invention, comprising at least two first components 21, 41 of different dimensions and/or at least two second components 22, 42 of different dimensions.

The kit 14 could, for example, comprise a tibial unit 2 provided with a first small tibial component 21L0, a first medium tibial component 21L1 and a first large tibial component 21L2, as well as a second small tibial component 22S, a second medium tibial component 22M and a second large tibial component 22L. The first tibial components 21L0, 21L1, 21L2 are usable in combination with any one of the second tibial components 22S, 22M and 22L depending on the needs.

In alternative or in addition to this, the kit 14 could, for example, comprise a femoral unit 4 provided with a first small femoral component 41L0, a first medium femoral component 41L1 and a first large femoral component 41L2, as well as a second small femoral component 42S, a second medium femoral component 42M and a second large femoral component 42L. The first femoral components 41L0, 41L1, 41L2 are usable in combination with any one of the second femoral components 42S, 42M and 42L depending on needs.

To facilitate modularity in a kit according to the present invention, the raised intermediate parts 2aa of all the second tibial components 22 have the same dimensions and the hollow central bands 4aa of all the second femoral components 44 have the same dimensions.

Such modularity of the spacer device 1 allows adapting the latter to the anthropomorphic measurements of the femoral and tibial ends of a patient.

For implanting in a knee a spacer device with a method according to the present invention, the surgeon first decides which components to use, after which the first tibial component 21 or the first stem 21b of the latter is implanted in the tibia and the first femoral component 41 or the second stem 41b of the same in the femur, and therefore the fixing means are applied, for example, an adhesive layer is applied, such as a layer of bone cement on the second intermediate surface 2b and on the second intermediate face 4b. At this point, with the fixing means in the first rest condition, for example before the curing or polymerisation of the adhesive layers, the second tibial component 22 is arranged on the first tibial component 21 and, more particularly, the fourth intermediate surface 2d on the second intermediate surface 2b as well as the second femoral component 42 in abutment against the first tibial component 41 and, more particularly, the fourth intermediate face 4d against the second intermediate face 4b.

Subsequently, and still before the curing or polymerisation of the adhesive layers, the tibia and femur are mutually displaced so as to reduce the joint, that is to say so as to straighten, in fact, the leg and bring the first joint face 4a within the sliding seat 3 for the sliding engagement of the first joint surface 2a.

In this way, the tibial unit 2 and the femoral unit 4 are brought in interference and, thanks to the fact that the adhesive layer is not yet polymerised, in the case wherein reciprocal tensions or stresses between the units 2 and 4 remain, the latter, in fact, self-centre or otherwise the relative position of the same is adjusted automatically, thus obtaining in this way a physiological collimation or a natural alignment neutralising, in fact, the risk of wrong placements, which occur very frequently when using traditional spacers which present medium dimensions and are little anatomical. Then, when the fixing means are brought into the second position, for example during the curing of the adhesive layers, the spacer device is properly implanted. In this regard, thanks to the structures of the tibial 2 and femoral 4 units of a device according to the present invention, it is possible to obtain a self-adjustment of the relative position of the same along three different axes or directions, that is to say a first front F-rear R direction, a second direction from one side S1 to the other S2 and/or a third direction orthogonal to the first and second direction, that is to say a direction orthogonal to the intermediate surfaces 2b, 2d along which the two tibial 2 and femoral 4 units, and hence the first joint surface 2a and the first joint face 4a, are moved away or approached.

Moreover, as mentioned above, one could alternatively have only the tibial unit 2 or the femoral unit 4 in two components, in which case it would still be possible to obtain a self-adjustment of the device when straightening the leg.

As will be understood, a spacer device according to the present invention allows for an optimal adjustment or adaptation to the respective implant area and may be implanted in a very quick and easy manner.

As regards WO2013041905A1, the respective spacer does neither provide a tibial unit and/or femoral unit with two components, nor, indeed, provided with two components adjustably fixable as in a spacer according to the present invention.

Furthermore, according to this international application the rod-like element of the tibial component is set up to determine a correct relative positioning between the tibial component and the shim, so as to ensure the "runout", i. e. the alignment between main axis of the rod-like element of the component tibial and the longitudinal axis of symmetry of the hole delimited by the shim. It is therefore not possible to fix in a regulated manner in a plurality of operational positions the tibial component and the shim.

It should also be taken into account that in WO2013041905A1 it is also stated that some lower lugs of the tibial component are, in use, in abutment on respective protuberances of the shim, and this confirms that this prior art document does not provide any adjustability of the relative position between the tibial component and the shim.

Modifications and variations of the invention are possible within the scope of protection defined by the claims.

## Claims

1. A spacer device for knee intended to be temporarily implanted at the joint area between the tibia and femur of a patient for the replacement of an infected joint prosthesis and for the preservation of size or spaces of the patient joint area prior to the implantation of a new prosthesis, said spacer device comprising:
- a tibial unit (2) intended to be fixed to the tibia of a patient and having an upper, in use, first joint surface (2a) substantially curved and delimiting a substantially concave sliding seat (3), as well as
- a femoral unit (4) intended to be fixed to the femur of a patient and having a lower, in use, first joint face (4a), substantially convex and intended to be positioned in said sliding seat (3) for the sliding engagement with said first upper joint surface (2a), thereby allowing the mutual angular displacement between said tibial unit (2) and said femoral unit (4),
wherein said tibial unit (2) comprises:
- a first component (21) fixable to the tibia and distal from said first joint surface (2a), as well as
- a second component (22) adjustably fixable to the first component (21) in a plurality of operational positions and delimiting said first joint surface (2a), wherein said second component (22) is adjustably fixable to the first component (21) in a plurality of operational positions from the front (F) to the rear (R) along a first sagittal direction of said device and/or from one side (S1) to the other side (S2) along a second transverse direction of said device.

2. A spacer device for knee intended to be temporarily implanted at the joint area between the tibia and femur of a patient for the replacement of an infected joint prosthesis and for the preservation of size or spaces of the patient joint area prior to the implantation of a new prosthesis, said spacer device comprising:
- a tibial unit (2) intended to be fixed to the tibia of a patient and having an upper, in use, first joint surface (2a) substantially curved and delimiting a substantially concave sliding seat (3), as well as
- a femoral unit (4) intended to be fixed to the femur of a patient and having a lower, in use, first joint face (4a), substantially convex and intended to be positioned in said sliding seat (3) for the sliding engagement with said first upper joint surface (2a), thereby allowing the mutual angular displacement between said tibial unit (2) and said femoral unit (4),
wherein said femoral unit (4) comprises:
- a first component (41) fixable to the femur and distal from said first joint face (4a), as well as
- a second component (42) adjustably fixable to the first component (41) in a plurality of operational positions and delimiting said first joint face (4a).

3. A device according to claim 1 or 2, comprising fixing means between said first component (21, 41) and said second component (22, 42) having a first rest position or condition, in which said fixing means connect without fixing said first component (21, 41) and said second component (22, 42), and a second fixing position or condition, in which said fixing means fix said second component (22, 42) to said first component (21, 41) in one position of said plurality of operational positions.

4. A device according to claim 3, wherein said fixing means comprise at least one adhesive layer between said first component (21, 41) and said second component (22, 42) having a time of curing or solidifying, said at least one adhesive layer being intended initially to grip or adhere on one side with said first component (21, 41) and on the other with said second component (22, 42), thereby connecting without fixing said first (21, 41) and said second component (22, 42), as well as to subsequently harden or solidify so as to fix said second component (22, 42) in one position of said plurality of operational positions, so that it is possible to adjust the relative position between said first (21, 41) and said second (22, 42) component during the time of curing or solidifying of said at least one adhesive layer.

5. A device according to claim 3 or 4, wherein said at least one adhesive layer comprises a layer of bone cement.

6. A device according to any one of the preceding claims, wherein said second component (22, 42) is adjustably fixable to the first component (21, 41) in a plurality of operational positions along a first sagittal direction, i.e. from the front (F) to the rear (R) of said device, along a second transverse direction, i.e. from one side (S1) to the other side (S2) of said device and/or along a longitudinal direction or direction orthogonal to said first and said second direction.

7. A device according to any one of the preceding claims, wherein:
- said tibial unit (2) comprises a first tibial component (21) fixable to the tibia and distal from said first joint surface (2a) as well as a second tibial component (22) adjustably fixable to said first tibial component (21) in a plurality of operational positions and delimiting said first joint surface (2a), respectively, while
- said femoral unit (4) comprises a first femoral component (41) fixable to the femur and distal from said first joint face (4a) as well as a second femoral component (42) adjustably fixable to said first femoral component (41) in a plurality of operational positions and delimiting said first joint face (4a), respectively.

8. A device according to any one of the preceding claims, wherein said tibial unit (2) comprises a first tibial component (21) fixable to the tibia as well as a second tibial component (22) adjustably fixable to the first tibial component (21) and wherein said first tibial component (21) has a base portion (21a) delimiting a second intermediate surface (2b) facing towards said second tibial component (22) and a third connection surface (2c) opposite to said second intermediate surface (2b) and further comprises a first stem (21b) protruding from said third connection surface (2c) of said base portion (21a) and intended to be implanted in the tibia of a patient, while said second tibial component (22) on one side delimits said first upper joint surface (2a) and on the other side a fourth intermediate surface (2d) intended to be abutted against said second intermediate surface (2b) of said first tibial component (21).

9. A device according to claim 8, wherein said base portion (21a) of said first tibial component (21) is substantially C-shaped and delimits a first channel (6) extending from said second intermediate surface (2b) to said third connection surface (2c), while said second tibial component (22) is substantially C-shaped and delimits a second channel (7) extending from said first joint surface (2a) to said fourth intermediate surface (2d) and is substantially aligned with said first channel (6).

10. A device according to claim 4 and according to claim 8 or 9, wherein said second intermediate surface (2b) of said first tibial component (21) or said fourth intermediate surface (2d) of said second tibial component (22) has at least one rough or jagged area (8) and wherein said at least one adhesive layer is placed at said at least one rough or jagged area (8).

11. A device according to any one of the preceding claims, comprising reference means for the guided positioning with unstable constraint (9a, 9b) of said second component (22, 42) on the respective first component (21, 41).

12. A device according to claim 11, wherein said guided positioning means with unstable constraint (9a, 9b) comprise at least one pin component (9a) and at least one guided positioning recess (9b) of said pin component (9a), and wherein said tibial unit (2) comprises the first tibial component (21) fixable to the tibia as well as the second tibial component (22) adjustably fixable to the first tibial component (21) and wherein starting from said first tibial component (21) or from said second tibial component (22) said at least one pin component (9a) extends, while in the other between said second tibial component (22) and said first tibial component (21) said at least one positioning recess (9b) is formed, said pin component (9a) having a width smaller than said positioning recess (9b), so that the engagement between said pin component (9a) and said positioning recess (9b) is loose.

13. A device according to any one of the preceding claims, wherein said femoral unit (4) comprises the first femoral component (41) fixable to the femur as well as the second femoral component (42) adjustably fixable to the first femoral component (41) and wherein said first femoral component (41) has a main part (41a) delimiting a second intermediate face (4b) facing towards said second femoral component (42) and a third connection face (4c) opposite to said second intermediate face (4b) and further comprises a second stem (41b) extending from said third connection face (4c) of said main part (41a) and intended to be implanted in the femur of a patient, while said second femoral component (42) on one side delimits said first lower joint face (4a) and on the other side delimits a fourth intermediate face (4d) intended to be abutted against said second intermediate face (4b) of said first femoral component (41).

14. A device according to claim 13, wherein said main part (41a) of said first femoral component (41) is substantially C-shaped and delimits a third channel (10) extending from said second intermediate face (4b) to said third connection face (4c), while said second femoral component (42) is substantially C-shaped and delimits a fourth channel (11) extending from said first joint face (4a) to said fourth intermediate face (4d) and is substantially aligned with said third channel (10).

15. A device according to any one of claims 13 or 14 when claim 13 or 14 depends upon claim 4, wherein said second intermediate face (4b) of said first femoral component (41) or said fourth intermediate face (4d) of said second femoral component (42) has at least one rough or jagged area (12) and wherein said at least one adhesive layer is placed at said at least one rough or jagged area (12).

16. A device according to any one of the preceding claims, wherein at least one of said tibial unit (2) and said femoral unit (4) is made of a biologically compatible material, added and/or additivable with one or more pharmaceutical products, active and/or therapeutic ingredients designed to be released in said joint area of a patient so as to treat the infection at the tibia and femur bone ends.

17. A kit for the construction of a spacer device for knee according to any one of the preceding claims, comprising at least two first components (21, 41) with size different from one another and/or at least two seconds components (22, 42) with size different from one another.

## Patentansprüche

1. Abstandsvorrichtung für das Knie, die dazu bestimmt ist, vorübergehend in den Gelenkbereich zwischen der Tibia und dem Femur eines Patienten implantiert zu werden, um eine infizierte Gelenkprothese zu ersetzen und um die Größe oder die Räume des Patientengelenkbereichs vor der Implantation einer neuen Prothese zu erhalten, wobei die besagte Abstandsvorrichtung umfasst:
- eine Tibia-Einheit (2), die dazu bestimmt ist, an der Tibia eines Patienten befestigt zu werden, und eine obere, bei Verwendung, erste Gelenkoberfläche (2a) aufweist, die im Wesentlichen gekrümmt ist und einen im Wesentlichen konkaven Gleitsitz (3) begrenzt, sowie
- eine Femur-Einheit (4), die dazu bestimmt ist, am Femur eines Patienten befestigt zu werden, und eine untere, bei Verwendung, erste Gelenkfläche (4a) aufweist, die im Wesentlichen konvex ist und dazu bestimmt ist, in dem besagten Gleitsitz (3) für den gleitenden Eingriff mit der besagten ersten oberen Gelenkoberfläche (2a) positioniert zu werden, wodurch die gegenseitige Winkelverschiebung zwischen der besagten Tibia-Einheit (2) und der besagten Femur-Einheit (4) ermöglicht wird,
wobei die besagte Tibia-Einheit (2) umfasst:
- eine erste Komponente (21), die an der Tibia und distal von der besagten ersten Gelenkoberfläche (2a) fixierbar ist, sowie
- eine zweite Komponente (22), die in einer Vielzahl von Einsatzpositionen an der ersten Komponente (21) einstellbar fixierbar ist und die besagte erste Gelenkoberfläche (2a) begrenzt, worin die besagte zweite Komponente (22) an der ersten Komponente (21) in einer Vielzahl von Einsatzpositionen von der Vorderseite (F) zur Rückseite (R) entlang einer ersten sagittalen Richtung der besagten Vorrichtung und/oder von einer Seite (S1) zur anderen Seite (S2) entlang einer zweiten Querrichtung der besagten Vorrichtung einstellbar fixierbar ist.

2. Abstandsvorrichtung für das Knie, die dazu bestimmt ist, vorübergehend in den Gelenkbereich zwischen der Tibia und dem Femur eines Patienten implantiert zu werden, um eine infizierte Gelenkprothese zu ersetzen und um die Größe oder die Räume des Patientengelenkbereichs vor der Implantation einer neuen Prothese zu erhalten, wobei die besagte Abstandsvorrichtung umfasst:
- eine Tibia-Einheit (2), die dazu bestimmt ist, an der Tibia eines Patienten befestigt zu werden, und eine obere, bei Verwendung, erste Gelenkoberfläche (2a) aufweist, die im Wesentlichen gekrümmt ist und einen im Wesentlichen konkaven Gleitsitz (3) begrenzt, sowie
- eine Femur-Einheit (4), die dazu bestimmt ist, am Femur eines Patienten befestigt zu werden, und eine untere, bei Verwendung, erste Gelenkfläche (4a) aufweist, die im Wesentlichen konvex ist und dazu bestimmt ist, in dem besagten Gleitsitz (3) für den gleitenden Eingriff mit der besagten ersten oberen Gelenkoberfläche (2a) positioniert zu werden, wodurch die gegenseitige Winkelverschiebung zwischen der besagten Tibia-Einheit (2) und der besagten Femur-Einheit (4) ermöglicht wird,
wobei die besagte Femur-Einheit (4) umfasst:
- eine erste Komponente (41), die am Femur und distal von der besagten ersten Gelenkfläche (4a) fixierbar ist, sowie
- eine zweite Komponente (42), die in einer Vielzahl von Einsatzpositionen an der ersten Komponente (41) einstellbar fixierbar ist und die besagte erste Gelenkfläche (4a) begrenzt.

3. Vorrichtung nach Anspruch 1 oder 2, umfassend Befestigungsmittel zwischen der besagten ersten Komponente (21, 41) und der besagten zweiten Komponente (22, 42) mit einer ersten Ruheposition oder -bedingung, in der die besagten Befestigungsmittel ohne Fixierung die besagte erste Komponente (21, 41) und die besagte zweite Komponente (22, 42) verbinden, und eine zweite Befestigungsposition oder -bedingung, in der die besagten Befestigungsmittel die besagte zweite Komponente (22, 42) mit der besagten ersten Komponente (21, 41) in einer Position der besagten Vielzahl von Einsatzpositionen fixieren.

4. Vorrichtung nach Anspruch 3, worin die besagten Befestigungsmittel mindestens eine Klebeschicht zwischen der besagten ersten Komponente (21, 41) und der besagten zweiten Komponente (22, 42) mit einer Zeit des Aushärtens oder Verfestigens umfassen, wobei die besagte mindestens eine Klebeschicht zunächst zum Haften oder Kleben auf einer Seite mit der besagten ersten Komponente (21, 41) und auf der anderen mit der besagten zweiten Komponente (22, 42) bestimmt ist, wodurch die besagte erste (21, 41) und die besagte zweite Komponente (22, 42) ohne Fixierung verbunden werden, sowie anschließend zum Härten oder zum Verfestigen, um die besagte zweite Komponente (22, 42) in einer Position der besagten Vielzahl von Einsatzpositionen zu fixieren, sodass es möglich ist, die relative Position zwischen der besagten ersten (21, 41) und der besagten zweiten (22, 42) Komponente während der Zeit des Aushärtens oder Verfestigens der besagten mindestens einen Klebeschicht einzustellen.

5. Vorrichtung nach Anspruch 3 oder 4, worin die besagte mindestens eine Klebeschicht eine Schicht aus Knochenzement umfasst.

6. Vorrichtung nach irgendeinem der vorangegangenen Ansprüche, worin die besagte zweite Komponente (22, 42) an der ersten Komponente (21, 41) in einer Vielzahl von Einsatzpositionen entlang einer ersten sagittalen Richtung, d.h. von der Vorderseite (F) zur Rückseite (R) der besagten Vorrichtung, entlang einer zweiten Querrichtung, d.h. von einer Seite (S1) zur anderen Seite (S2) der besagten Vorrichtung und/oder entlang einer Längsrichtung oder einer Richtung orthogonal zu der besagten ersten und zweiten Richtung, einstellbar fixierbar ist.

7. Vorrichtung nach irgendeinem der vorangegangenen Ansprüche, worin:
- die besagte Tibia-Einheit (2) eine erste Tibiakomponente (21) umfasst, die an der Tibia und distal von der besagten ersten Gelenkfläche (2a) fixierbar ist, sowie eine zweite Tibiakomponente (22), die an der besagten ersten Tibiakomponente (22) in einer Vielzahl von Einsatzpositionen einstellbar fixierbar ist und jeweils die besagte erste Gelenkfläche (2a) begrenzt, während
- die besagte Femur-Einheit (4) eine erste Femurkomponente (41) umfasst, die am Femur und distal von der besagten ersten Gelenkfläche (4a) fixierbar ist, sowie eine zweite Femurkomponente (42), die an der besagten ersten Femurkomponente (41) in einer Vielzahl von Einsatzpositionen einstellbar fixierbar ist und jeweils die besagte erste Gelenkfläche (4a) begrenzt.

8. Vorrichtung nach irgendeinem der vorangegangenen Ansprüche, worin die besagte Tibia-Einheit (2) die erste Tibiakomponente (21), die an der Tibia fixierbar ist, sowie die zweite Tibiakomponente (22), die an der ersten Tibiakomponente (21) einstellbar fixierbar ist, umfasst und worin die besagte erste Tibiakomponente (21) einen Basisabschnitt (21a) aufweist, der eine zweite Zwischenfläche begrenzt (2b), die der besagten zweiten Tibiakomponente (22) und einer dritten Verbindungsfläche (2c) gegenüber der besagten zweiten Zwischenfläche (2b) zugewandt ist und ferner einen ersten Schaft (21b) umfasst, der sich von der besagten dritten Verbindungsfläche (2c) des besagten Basisabschnitts (21a) erstreckt und dazu bestimmt ist, in die Tibia eines Patienten implantiert zu werden, während die besagte zweite Tibiakomponente (22) auf einer Seite die besagte erste obere Gelenkoberfläche (2a) begrenzt und auf der anderen Seite eine vierte Zwischenfläche (2d) begrenzt, die dazu bestimmt ist, an der besagten zweiten Zwischenfläche (2b) der besagten ersten Tibiakomponente (21) in Anlage gebracht zu werden.

9. Vorrichtung nach Anspruch 8, worin der besagte Basisabschnitt (21a) der besagten ersten Tibiakomponente (21) im Wesentlichen C-förmig ist und einen ersten Kanal (6) begrenzt, der sich von der besagten zweiten Zwischenfläche (2b) zu der besagten dritten Verbindungsfläche (2c) erstreckt, während die besagte zweite Tibiakomponente (22) im Wesentlichen C-förmig ist und einen zweiten Kanal (7) begrenzt, der sich von der besagten ersten Gelenkfläche (2a) zu der besagten vierten Zwischenfläche (2d) erstreckt und im Wesentlichen mit dem besagten ersten Kanal (6) ausgerichtet ist.

10. Vorrichtung nach Anspruch 4 und nach Anspruch 8 oder 9, worin die besagte zweite Zwischenfläche (2b) der besagten ersten Tibiakomponente (21) oder die besagte vierte Zwischenfläche (2d) der besagten zweiten Tibiakomponente (22) mindestens einen rauen oder gezackten Bereich (8) aufweist und worin die besagte mindestens eine Klebeschicht auf dem besagten mindestens einen rauen oder gezackten Bereich (8) angeordnet ist.

11. Vorrichtung nach irgendeinem der vorangegangenen Ansprüche, umfassend Referenzmittel für die geführte Positionierung mit instabiler Festhaltung (9a, 9b) der besagten zweiten Komponente (22, 42) auf der jeweiligen ersten Komponente (21, 41).

12. Vorrichtung nach Anspruch 11, worin die besagten geführten Positioniermittel mit instabiler Festhaltung (9a, 9b) mindestens eine Stiftkomponente (9a) und mindestens eine geführte Positionieraussparung (9b) der besagten Stiftkomponente (9a) umfassen, und worin die besagte Tibia-Einheit (2) die erste Tibiakomponente (21) umfasst, die an der Tibia fixierbar ist, sowie die zweite Tibiakomponente (22), die an der ersten Tibiakomponente (21) einstellbar fixierbar ist, und worin sich ausgehend von der besagten ersten Tibiakomponente (21) oder der besagten zweiten Tibiakomponente (22) die mindestens eine Stiftkomponente (9a) erstreckt, während in der anderen zwischen der besagten zweiten Tibiakomponente (22) und der besagten ersten Tibiakomponente (21) die besagte mindestens eine Positionieraussparung (9b) ausgebildet ist, wobei die besagte Stiftkomponente (9a) eine Breite aufweist, die kleiner als die besagte Positionieraussparung (9b) ist, sodass der Eingriff zwischen der besagten Stiftkomponente (9a) und der besagten Positionieraussparung (9b) lose ist.

13. Vorrichtung nach irgendeinem der vorangegangenen Ansprüche, worin die besagte Femur-Einheit (4) die erste Femurkomponente (41), die am Femur fixierbar ist, sowie die zweite Femurkomponente (42), die an der ersten Femurkomponente (41) einstellbar fixierbar ist, umfasst und worin die besagte erste Femurkomponente (41) einen Hauptteil (41a) aufweist, der eine zweite Zwischenfläche begrenzt (4b), die der besagten zweiten Femurkomponente (42) und einer dritten Verbindungsfläche (4c) gegenüber der besagten zweiten Zwischenfläche (4b) zugewandt ist und ferner einen zweiten Schaft (41b) umfasst, der sich von der besagten dritten Verbindungsfläche (4c) des besagten Hauptteils (41a) erstreckt und dazu bestimmt ist, in das Femur eines Patienten implantiert zu werden, während die besagte zweite Femurkomponente (42) auf einer Seite die besagte erste untere Gelenkoberfläche (4a) begrenzt und auf der anderen Seite eine vierte Zwischenfläche (4d) begrenzt, die dazu bestimmt ist, an der besagten zweiten Zwischenfläche (4b) der besagten ersten Femurkomponente (41) in Anlage gebracht zu werden.

14. Vorrichtung nach Anspruch 13, worin der besagte Hauptteil (41a) der besagten ersten Femurkomponente (41) im Wesentlichen C-förmig ist und einen dritten Kanal (10) begrenzt, der sich von der besagten zweiten Zwischenfläche (4b) zu der besagten dritten Verbindungsfläche (4c) erstreckt, während die besagte zweite Femurkomponente (42) im Wesentlichen C-förmig ist und einen vierten Kanal (11) begrenzt, der sich von der besagten ersten Gelenkfläche (4a) zu der besagten vierten Zwischenfläche (4d) erstreckt und im Wesentlichen mit dem besagten dritten Kanal (10) ausgerichtet ist.

15. Vorrichtung nach irgendeinem der Ansprüche 13 oder 14, wenn Anspruch 13 oder 14 von Anspruch 4 abhängt, worin die besagte zweite Zwischenfläche (4b) der besagten ersten Femurkomponente (41) oder die besagte vierte Zwischenfläche (4d) der besagten zweiten Femurkomponente (42) mindestens einen rauen oder gezackten Bereich (12) aufweist und worin die besagte mindestens eine Klebeschicht auf dem besagten mindestens einen rauen oder gezackten Bereich (12) angeordnet ist.

16. Vorrichtung nach irgendeinem der vorangegangenen Ansprüche, worin mindestens eine der besagten Tibia-Einheit (2) und der besagten Femur-Einheit (4) aus einem biologisch verträglichen Material hergestellt, das mit einem oder mehreren pharmazeutischen Produkten, aktiven und/oder therapeutischen Bestandteilen, ergänzt oder additivierbar ist, die dazu bestimmt sind, in dem besagten Gelenkbereich eines Patienten freigesetzt zu werden, um die Infektion an den Tibia- und Femurenden zu behandeln.

17. Kit für die Konstruktion einer Abstandsvorrichtung für das Knie nach irgendeinem der vorangegangenen Ansprüche, umfassend mindestens zwei erste Komponenten (21, 41) mit voneinander verschiedener Größe und/oder mindestens zwei zweite Komponenten (22, 42) mit voneinander verschiedener Größe.

## Revendications

1. Dispositif d'espacement pour genou destiné à être temporairement implanté dans la zone de l'articulation entre le tibia et le fémur d'un patient pour le remplacement d'une prothèse d'articulation infectée et pour le maintien de la taille ou des espaces de la zone de l'articulation du patient avant l'implantation d'une nouvelle prothèse, ledit dispositif d'espacement comprenant :
- une unité tibiale (2) destinée à être fixée au tibia d'un patient et comportant, pendant l'utilisation, une première surface d'articulation supérieure (2a) sensiblement incurvée et délimitant un siège coulissant sensiblement concave (3), ainsi que
- une unité fémorale (4) destinée à être fixée au fémur d'un patient et comportant, pendant l'utilisation, une première face d'articulation inférieure (4a), sensiblement convexe et destinée à être positionnée dans ledit siège coulissant (3) pour l'accouplement coulissant avec ladite première surface d'articulation supérieure (2a), permettant ainsi le déplacement angulaire mutuel entre ladite unité tibiale (2) et ladite unité fémorale (4),
dans lequel ladite unité tibiale (2) comprend :
- un premier composant (21) fixable au tibia et distal de ladite première surface d'articulation (2a), ainsi que
- un deuxième composant (22) fixable de manière ajustable au premier composant (21) dans une pluralité de positions d'utilisation et délimitant ladite première surface d'articulation (2a), dans lequel ledit deuxième composant (22) est fixable de manière ajustable au premier composant (21) dans une pluralité de positions d'utilisation de l'avant (F) à l'arrière (R) le long d'une première direction sagittale dudit dispositif et/ou d'un côté (S1) à l'autre côté (S2) le long d'une deuxième direction transversale dudit dispositif.

2. Dispositif d'espacement pour genou destiné à être temporairement implanté dans la zone de l'articulation entre le tibia et le fémur d'un patient pour le remplacement d'une prothèse d'articulation infectée et pour le maintien de la taille ou des espaces de la zone de l'articulation du patient avant l'implantation d'une nouvelle prothèse, ledit dispositif d'espacement comprenant :
- une unité tibiale (2) destinée à être fixée au tibia d'un patient et comportant, pendant l'utilisation, une première surface d'articulation supérieure (2a) sensiblement incurvée et délimitant un siège coulissant sensiblement concave (3), ainsi que
- une unité fémorale (4) destinée à être fixée au fémur d'un patient et comportant, pendant l'utilisation, une première face d'articulation inférieure (4a), sensiblement convexe et destinée à être positionnée dans ledit siège coulissant (3) pour l'accouplement coulissant avec ladite première surface d'articulation supérieure (2a), permettant ainsi le déplacement angulaire mutuel entre ladite unité tibiale (2) et ladite unité fémorale (4),
dans lequel ladite unité fémorale (4) comprend :
- un premier composant (41) fixable au fémur et distal de ladite première face d'articulation (4a), ainsi que
- un deuxième composant (42) fixable de manière ajustable au premier composant (41) dans une pluralité de positions d'utilisation et délimitant ladite première face d'articulation (4a).

3. Dispositif selon la revendication 1 ou 2, comprenant des moyens de fixation entre ledit premier composant (21, 41) et ledit deuxième composant (22, 42) ayant une première position ou condition de repos, dans laquelle lesdits moyens de fixation raccordent sans fixation ledit premier composant (21, 41) et ledit deuxième composant (22, 42), et une deuxième position ou condition de fixation, dans laquelle lesdits moyens de fixation fixent ledit deuxième composant (22, 42) audit premier composant (21, 41) dans une position de ladite pluralité de positions d'utilisation.

4. Dispositif selon la revendication 3, dans lequel lesdits moyens de fixation comprennent au moins une couche adhésive entre ledit premier composant (21, 41) et ledit deuxième composant (22, 42) ayant une durée de cuisson ou de solidification, ladite au moins une couche adhésive étant destinée initialement à s'accrocher ou adhérer sur un côté avec ledit premier composant (21, 41) et sur l'autre avec ledit deuxième composant (22, 42), raccordant ainsi sans fixer ledit premier (21, 41) et ledit deuxième composant (22, 42), ainsi qu'à durcir ou se solidifier ensuite de façon à fixer ledit deuxième composant (22, 42) dans une position de ladite pluralité de positions d'utilisation, de telle sorte qu'il soit possible d'ajuster la position relative entre ledit premier (21, 41) et ledit deuxième (22, 42) composant pendant la durée de cuisson ou de solidification de ladite au moins une couche adhésive.

5. Dispositif selon la revendication 3 ou 4, dans lequel ladite au moins une couche adhésive comprend une couche de ciment osseux.

6. Dispositif selon l'une quelconque des revendications précédentes, dans lequel ledit deuxième composant (22, 42) est fixable de manière ajustable au premier composant (21, 41) dans une pluralité de positions d'utilisation le long d'une première direction sagittale, c'est-à-dire de l'avant (F) à l'arrière (R) dudit dispositif, le long d'une deuxième direction transversale, c'est-à-dire d'un côté (S1) à l'autre côté (S2) dudit dispositif et/ou le long d'une direction longitudinale ou d'une direction orthogonale à ladite première et ladite deuxième direction.

7. Dispositif selon l'une quelconque des revendications précédentes, dans lequel :
- ladite unité tibiale (2) comprend un premier composant tibial (21) fixable au tibia et distal de ladite première surface d'articulation (2a) ainsi qu'un deuxième composant tibial (22) fixable de manière ajustable audit premier composant tibial (21) dans une pluralité de positions d'utilisation et délimitant ladite première surface d'articulation (2a), respectivement, alors que
- ladite unité fémorale (4) comprend un premier composant fémoral (41) fixable au fémur et distal de ladite première face d'articulation (4a) ainsi qu'un deuxième composant fémoral (42) fixable de manière ajustable audit premier composant fémoral (41) dans une pluralité de positions d'utilisation et délimitant ladite première face d'articulation (4a), respectivement.

8. Dispositif selon l'une quelconque des revendications précédentes, dans lequel ladite unité tibiale (2) comprend un premier composant tibial (21) fixable au tibia ainsi qu'un deuxième composant tibial (22) fixable de manière ajustable au premier composant tibial (21) et dans lequel ledit premier composant tibial (21) comporte une partie de base (21a) délimitant une deuxième surface intermédiaire (2b) orientée vers ledit deuxième composant tibial (22) et une troisième surface de raccordement (2c) opposée à ladite deuxième surface intermédiaire (2b) et comprend en outre une première tige (21b) saillante depuis ladite troisième surface de raccordement (2c) de ladite partie de base (21a) et destinée à être implantée dans le tibia d'un patient, alors que ledit deuxième composant tibial (22) délimite sur un côté ladite première surface d'articulation supérieure (2a) et sur l'autre côté une quatrième surface intermédiaire (2d) destinée à être en butée contre ladite deuxième surface intermédiaire (2b) dudit premier composant tibial (21).

9. Dispositif selon la revendication 8, dans lequel ladite partie de base (21a) dudit premier composant tibial (21) est sensiblement en C et délimite un premier canal (6) s'étendant de ladite deuxième surface intermédiaire (2b) à ladite troisième surface de raccordement (2c), alors que ledit deuxième composant tibial (22) est sensiblement en C et délimite un deuxième canal (7) s'étendant de ladite première surface d'articulation (2a) à ladite quatrième surface intermédiaire (2d) et est sensiblement aligné avec ledit premier canal (6).

10. Dispositif selon la revendication 4 et selon la revendication 8 ou 9, dans lequel ladite deuxième surface intermédiaire (2b) dudit premier composant tibial (21) ou ladite quatrième surface intermédiaire (2d) dudit deuxième composant tibial (22) comporte au moins une zone rugueuse ou dentelée (8) et dans lequel ladite au moins une couche adhésive est placée sur ladite au moins une zone rugueuse ou dentelée (8).

11. Dispositif selon l'une quelconque des revendications précédentes, comprenant des moyens de référence pour le positionnement guidé avec une contrainte instable (9a, 9b) dudit deuxième composant (22, 42) sur le premier composant (21,41) respectif.

12. Dispositif selon la revendication 11, dans lequel lesdits moyens de positionnement guidé avec une contrainte instable (9a, 9b) comprennent au moins un composant d'axe (9a) et au moins un renfoncement de positionnement guidé (9b) dudit composant d'axe (9a), et dans lequel ladite unité tibiale (2) comprend le premier composant tibial (21) fixable au tibia ainsi que le deuxième composant tibial (22) fixable de manière ajustable au premier composant tibial (21) et dans lequel à partir dudit premier composant tibial (21) ou dudit deuxième composant tibial (22) ledit au moins un composant d'axe (9a) s'étend, alors que dans l'autre entre ledit deuxième composant tibial (22) et ledit premier composant tibial (21) ledit au moins un renfoncement de positionnement (9b) est formé, ledit composant d'axe (9a) ayant une largeur inférieure audit renfoncement de positionnement (9b), de telle sorte que l'accouplement entre ledit composant d'axe (9a) et ledit renfoncement de positionnement (9b) est lâche.

13. Dispositif selon l'une quelconque des revendications précédentes, dans lequel ladite unité fémorale (4) comprend le premier composant fémoral (41) fixable au fémur ainsi que le deuxième composant fémoral (42) fixable de manière ajustable au premier composant fémoral (41) et dans lequel ledit premier composant fémoral (41) comporte une partie principale (41a) délimitant une deuxième face intermédiaire (4b) orientée vers ledit deuxième composant fémoral (42) et une troisième face de raccordement (4c) opposée à ladite deuxième face intermédiaire (4b) et comprend en outre une deuxième tige (41b) s'étendant depuis ladite troisième face de raccordement (4c) de ladite partie principale (41a) et destinée à être implantée dans le fémur d'un patient, alors que ledit deuxième composant fémoral (42) délimite sur un côté ladite première face d'articulation inférieure (4a) et sur l'autre côté une quatrième face intermédiaire (4d) destinée à être en butée contre ladite deuxième face intermédiaire (4b) dudit premier composant fémoral (41).

14. Dispositif selon la revendication 13, dans lequel ladite partie principale (41a) dudit premier composant fémoral (41) est sensiblement en C et délimite un troisième canal (10) s'étendant de ladite deuxième face intermédiaire (4b) à ladite troisième face de raccordement (4c), alors que ledit deuxième composant fémoral (42) est sensiblement en C et délimite un quatrième canal (11) s'étendant de ladite première face d'articulation (4a) à ladite quatrième face intermédiaire (4d) et est sensiblement aligné avec ledit troisième canal (10).

15. Dispositif selon l'une quelconque des revendications 13 ou 14 lorsque la revendication 13 ou 14 dépend de la revendication 4, dans lequel ladite deuxième face intermédiaire (4b) dudit premier composant fémoral (41) ou ladite quatrième face intermédiaire (4d) dudit deuxième composant fémoral (42) comporte au moins une zone rugueuse ou dentelée (12) et dans lequel ladite au moins une couche adhésive est placée sur ladite au moins une zone rugueuse ou dentelée (12).

16. Dispositif selon l'une quelconque des revendications précédentes, dans lequel au moins l'une de ladite unité tibiale (2) et de ladite unité fémorale (4) est constituée d'un matériau biocompatible, ajouté et/ou additivable avec un ou plusieurs produits pharmaceutiques, ingrédients actifs et/ou thérapeutiques conçus pour être libérés dans ladite zone de l'articulation d'un patient de façon à traiter l'infection aux extrémités des os du tibia et du fémur.

17. Kit de construction d'un dispositif d'espacement pour genou selon l'une quelconque des revendications précédentes, comprenant au moins deux premiers composants (21, 41) de taille différente l'un de l'autre et/ou au moins deux deuxièmes composants (22, 42) de taille différente l'un de l'autre.
